# EUROPEAN PATENT APPLICATION

(11) **EP 4 696 779 A1**
(43) Date of publication of application: **18.02.2026**
(21) Application number: 24787738.4
(22) Date of filing: 09.01.2024
(51) Int. Cl.: C12N 9/64, C12N 1/19, C12N 1/38, C12R 1/84

(54) **BATROXOBIN FERMENTATION CULTURE MEDIUM AND FERMENTATION METHOD THEREFOR**

(30) Priority: 14.04.2023 CN 202310399752
(71) Applicant: Shanghai Tenry Pharmaceutical Co., Ltd., Fengxian District, Shanghai 201400 (CN)
(72) Inventor: LI, Qing, Shanghai 201400 (CN); CHEN, Wei, Shanghai 201400 (CN); ZHANG, Yan, Shanghai 201400 (CN); TANG, Fang, Shanghai 201400 (CN); GUAN, Lufan, Shanghai 201400 (CN); LI, Zhengwu, Shanghai 201400 (CN)
(74) Representative: ABG Intellectual Property Law, S.L.
(86) International application number: PCT/CN2024/071372
(87) International publication number: WO 2024/212631

(57) **Abstract**

Provided are a yeast fermentation method for recombinant batroxobin and a used culture medium. The fermentation steps mainly comprise primary seed culturing, secondary seed culturing and fermentation culturing. In the stage of fermentation culturing, by supplementing a plurality of nutrient components, such as glycerol, casamino acids and PTM1 inorganic salt, into a culture medium to optimize the culture medium, adjusting the fermentation culture medium to a proper pH value, and performing methanol induced culturing, the activity of the fermented recombinant protein is improved. The components of the optimized basic fermentation culture medium are: K₂SO₄: 0.91%, MgSO₄: 0.36%, CaSO₄•2H₂O: 0.059%, 85% H₃PO₄: 2.5% (V/V), KOH: 0.206%, glycerol: 4%, GPE: 0.05%-0.1%, PTM1: 0.4% (V/V), and casamino acids: 0.5%. The components of the optimized glycerol-supplement culture medium are: 50% glycerol + 12mL/L PTM1 + 0.5% casamino acids. The components of the optimized methanol induced culture medium are: 95% (100% methanol + 12mL/L PTM1) + 4.5% sorbitol + 0.5% casamino acids.

## Description

### Technical Field

The present invention relates to the field of bioengineering, specifically to a batroxobin fermentation culture medium and fermentation method therefor.

### Background Art

As early as 1963, Van Klobusitzky et al. purified an enzymatic hemostatic agent, Batroxobin, from the venom of Bothrops atrox, also known as "Batroxobin" in China. It is a single chain glycoprotein with a total of 231 amino acids and a molecular weight of 39-43 kDa. Batroxobin belongs to the serine protease family of molecules and is a single-chain glycoprotein that can specifically act on the Arg16-Gly17 peptide bond at the N-terminus of Aα chain of fibrinogen molecule, releasing fibrinopeptide A and generating unstable soluble fibrin I monomers, which then crosslink and polymerize into fibrin I polymers, promoting platelet aggregation at the bleeding site to produce hemostatic effects, and activating vascular endothelium to release tissue type plasminogen activator (t-PA), exerting antithrombotic effects.

With the increasing number of patients with thromboembolic diseases, the development of antithrombotic drugs has become one of the hotspots. On the one hand, molecular biology techniques are being used to engineer the molecular structure of existing drugs in order to obtain more effective thrombolytic drugs. On the other hand, there is an active search for some thrombolytic drugs from natural sources that are safe, effective, and have minimal side effects. It is reported that batroxobin has been widely used in clinical practice for the treatment of various diseases such as unstable angina and hyperviscosity syndrome, as well as the prevention of occlusive cardiovascular and cerebrovascular thrombotic diseases, with definite efficacy and minimal adverse reactions.

Since the large-scale production of batroxobin purified from snakes is limited, methods for producing recombinant proteins have been extensively studied by numerous researchers.

P. pastoris expression system combines the characteristics of clear genetic background, easy manipulation, and correct glycosylation in both prokaryotic and eukaryotic systems. Exogenous genes can be stably integrated into the yeast genome, efficiently expressing target proteins, and exogenous proteins can undergo protein post-translational modifications such as glycosylation; extracellular high secretion of target proteins is also beneficial for maintaining the biological activity and subsequent purification of the product, avoiding tedious steps such as prokaryotic expression and cell wall fragmentation, and improving yield. In 2004, You Weon-Kyoo from South Korea reported the expression of batroxobin in Pichia pastoris, with an expression level of 3.431 NIH/mL, and 7mg can be purified from per liter of fermentation broth.

Due to the structural characteristics of the batroxobin itself, the activity of batroxobin tends to decrease during the production process using genetic engineering fermentation. The main reason is that proteases in the yeast host system may degrade the accumulated batroxobin in the fermentation broth, thereby affecting the yield and enzyme activity of batroxobin. The genetic engineering expression of recombinant batroxobin is usually carried out using the Pichia pastoris system, but according to existing literatures, the expression level is not high.

Therefore, there are problems in the prior art such as low activity of batroxobin obtained by fermentation preparation and insufficient enzyme degradation.

### Summary of the Invention

The purpose of the present invention is to provide a batroxobin fermentation culture medium and fermentation method therefor directed to the problems in the prior art such as low yield and low enzyme activity of purified batroxobin.

The present invention discloses a culture medium for recombinant batroxobin fermentation, comprising: optimized basic fermentation culture medium, optimized glycerol-supplement culture medium, and optimized methanol induced culture medium, the components of the optimized basic fermentation culture medium are: K₂SO₄: 0.91%, MgSO₄: 0.36%, CaSO₄•2H₂O: 0.059%, 85% H₃PO₄: 2.5% (V/V), KOH: 0.206%, glycerol: 4%, GPE: 0.05%-0.1%, PTM1: 0.4% (V/V), and casamino acids: 0.5%; the components of the optimized glycerol-supplement culture medium are: 50% glycerol + 12mL/L PTM1 + 0.5% casamino acids; and the components of the optimized methanol induced culture medium are: 95% (100% methanol + 12mL/L PTM1) + 4.5% sorbitol + 0.5% casamino acids.

The present invention discloses a fermentation method for batroxobin, comprising the following steps:
1) Primary seed culturing, inoculating a preserved strain into a primary seed culture medium, wherein the formulation for the primary seed culture medium is yeast powder, 1.0%; polypeptone 2.0%; glucose, 2.0%, formulated with purified water and sterilized for future use;
2) Secondary seed culturing, under sterile conditions, inoculating the primary seed solution into a seed tank containing secondary culture medium, with an inoculation ratio of 5%-11%, the formulation for the secondary seed culture medium is yeast powder, 1.0%; polypeptone 2.0%; glucose, 2.0%, formulated with purified water and sterilized for future use;
3) Fermentation culturing, when the OD600 of the secondary seed solution is between 5-7, adding the optimized basic fermentation culture medium (4ml/L) to a fermentation tank under sterile conditions, transferring the secondary seed solution into the fermentation tank under sterile conditions, and after incubation for 4 hours, adding the optimized glycerol-supplement culture medium to the fermentation tank, adjusting pH value, and inducing with methanol; and
4) Collecting and purifying the supernatant to obtain recombinant batroxobin.

Preferably, the components of the optimized basic fermentation culture medium are: K₂SO₄: 0.91%, MgSO₄: 0.36%, CaSO₄•2H₂O: 0.059%, 85% H₃PO₄: 2.5% (V/V), KOH: 0.206%, glycerol: 4%, GPE: 0.05%-0.1%, PTM1: 0.4% (V/V), and casamino acids: 0.5%.

Preferably, the components of the optimized glycerol-supplement culture medium are: 50% glycerol + 12mL/L PTM1 + 0.5% casamino acids.

Preferably, during the methanol induction stage, the optimized methanol induced culture medium is used for induction, and the components of the optimized methanol induced culture medium are: 95% (100% methanol + 12mL/L PTM1) + 4.5% sorbitol + 0.5% casamino acids.

Preferably, the adjusted pH range is 5.0-7.0.

Preferably, the formulation of PTM1 is: 0.6% CuSO₄ ▪ 5H₂O, 0.008% NaI, 0.3% MnSO₄ ▪ H₂O, 0.02% Na₂MoO₄ ▪ 2H₂O, 0.02% H₃BO₃, 0.05% CoCl₂ ▪ 6H₂O, 6.5% FeSO₄ ▪ 7H₂O, 2% ZnCl₂, 0.02% Biotin, and 0.0005% (V/V) H₂SO₄.

Preferably, the optimized basic fermentation culture medium, the optimized glycerol-supplement culture medium, and the optimized methanol induced culture medium are separately formulated.

The present invention discloses a yeast fermentation method for recombinant batroxobin and a used culture medium, comprising the optimized basic fermentation culture medium, optimized glycerol-supplement culture medium, and optimized methanol induced culture medium. The fermentation steps mainly comprise primary seed culturing, secondary seed culturing, and fermentation culturing. In the stage of fermentation culturing, by supplementing a plurality of nutrient components, such as glycerol, casamino acids and PTM1 inorganic salt, into a culture medium, adjusting the fermentation culture medium to a proper pH value, and performing methanol induced culturing, the activity of the fermented recombinant protein is greatly improved. The present invention inhibits protease activity in fermentation products by adding casamino acids, and enhances the expression activity of the target protein by using sorbitol and methanol as co-carbon sources. After subsequent purification, , a highly active recombinant protein of batroxobin is prepared, which can achieve similar activity to commercially available natural batroxobin and is worthy of promoting and applying.

### Brief Description of the Drawings

Fig. 1. PCR screening and identification diagram of transformed yeast colonies.
Fig. 2. Activity determination of recombinant batroxobin at different stages of methanol induction.
Fig. 3. The effect of the components of the optimized basic culture medium on the activity of the fermented protein.
Fig. 4. The effect of the optimized methanol induced culture medium on the activity of the fermented protein.
Fig. 5. The effect of adding the glycerol-supplement culture medium on the activity of the fermented protein.
Fig. 6. The effect of the optimized glycerol-supplement culture medium on the activity of the fermented protein.
Fig. 7. Determination of the activity of fermented recombinant batroxobin at different pH values of the fermentation culture medium.
Fig. 8. SDS electrophoresis detection diagram of purified batroxobin protein.

### Detailed Description of Examples

The Detailed Description of Examples of the present invention will be described in detail below in combination with the Drawings. It should be understood that the Detailed Description of Examples described herein are only for illustrating and explaining the present invention, and are not intended to limit the present invention.

### Example 1. Construction and transformation of yeast expression vector containing batroxobin gene

### 1) Cloning of the gene encoding batroxobin

Using the nucleic acid sequence of Batroxobin (J02684.1) disclosed in GeneBank, the gene sequence was first artificially synthesized. Based on the information of the gene, the information about the enzyme cleavage sites in the gene, and the like, primer sequences for amplifying the gene encoding batroxobin were designed, and XhoI and SacII enzyme cleavage site sequences were added to both ends of the primers. The designed amplification primer sequences were as follows:
Sense primer: 5'- aactcgaggtcattgga ggtgatgaat -3';
Anti-sense primer: 5'- aaccgcggcgggcaagt cgcagttt -3'.

PCR amplification was performed on the synthesized batroxobin gene J02684.1 using the aforementioned amplification primers.

The PCR amplification reaction conditions were as follows:
95 °C for 3 min; 95 °C for 40s, 53 °C for 30s, 72 °C for 1 min, 35 cycles; and 72 °C for 15 min.

The obtained amplification product was stored at 4 °C for future use.

### 2) Construction and Transformation of Yeast Expression Vector

The PCR amplified product from step 1) was purified and recovered, and then double digested with XhoI and SacII, and at same time the yeast expression vector pPICZαA was also double digested with XhoI and SacII. After the digested products were recovered separately and then ligated with T4 ligase, and the ligated products were transformed into E. coli BL21. The recombinant bacteria were identified, the recombinant plasmid was extracted, and the positive clone was screened by double digestion and PCR amplification of the target gene, and the recombinant plasmid was named as pPICZ α A-Batr.

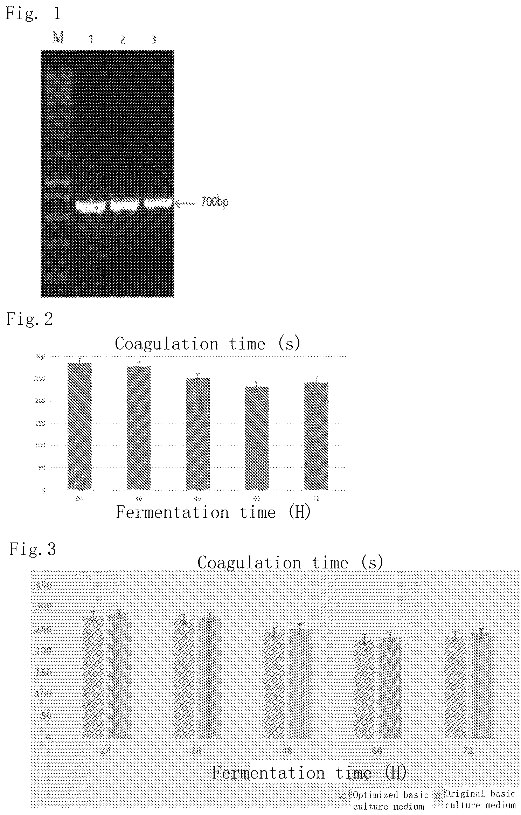

The pPICZαA-Batr plasmid was extracted, linearized with Sac I, and then transformed into Pichia pastoris X-33 by electrotransformation. All transformants were coated on YPD+Zeocin (300 µ g/ml) medium and incubated at 30 °C, the colonies were selected for colony PCR identification, and the above amplification primers were used for the PCR identification. Among them, the identification diagram of yeast colonies by PCR was shown in Fig. 1. The identified clones of the positive colony were stored for future use.

### Example 2. Fermentative expression of engineered yeast X-33/pPICZαA-Batr

### 1) Preliminary screening of enzyme expression levels in positive colonies

Positive clones were selected, first inoculated in glycerol composite medium (BMGY) and shaken overnight, then centrifuged to remove the culture medium. The precipitated bacteria were re-suspended in BMMY and diluted to OD₆₀₀ of 1 for induction of expression. Samples were taken every 12 hours and methanol was added to the final concentration of 1%, and the supernatant was collected by centrifugation after 72 h. Each sample was centrifuged and the supernatant was retained for preliminary determination of thrombin activity, the results of which were shown in Table 1. The clones of colonies with high thrombin activity were preliminarily screened and obtained for fermentation in a fermentation tank. The method for determining thrombin activity was as follows:
0.2ml of human-citrate coagulation quality control plasma was taken and added to a 96-well plate, incubated at 37 °C for 3 minutes. 0.2ml of sample solution preheated at 37 °C was added, immediately shaken to mix evenly, and timed, and the plasma coagulation condition was begun to check at 40s. The initial coagulation time was recorded, and 3 tubes were determined simultaneously. The error should be less than 20s. If the initial coagulation time was less than 40s, the concentration of the test sample solution that coagulated within (60 ± 20) seconds was recorded, when the test sample solution was diluted several times. Under the above conditions, the amount of enzyme that could coagulate 0.2ml of human-citrate anticoagulant plasma in 60s was defined as one enzyme activity unit.

**Table 1: Preliminary thrombin activity determination of the expression induced by different positive clone of engineered yeast X-33/pPICZαA-Batr for 36 hours**

| Colony Number | 1 | 2 | 3 | 4 | 5 | 6 |
|---|---|---|---|---|---|---|
| Coagulation time (S) | 262 | 231 | 247 | 289 | 255 | 269 |

It could be seen from the determination results that the activity of batroxobin expressed in colony 2 was the highest, and the coagulation time was the shortest. Colony 2 was used for later fermentation operation.

### Example 3. Optimization of small-scale fermentation conditions for yeast X-33/pPICZαA-Batr-2

### 1) Establishment of small-scale primary fermentation

① Primary seed culturing: 1-2 working seed strains were taken, opened under sterile conditions and inoculated into primary seed culture medium, with an inoculation ratio of 0.06% -0.2%, 30± 1 °C, 220rpm± 10rpm. The culturing was terminated when the OD₆₀₀ of the seed solution was 2-5, and the culture time was 30 ± 8h.

**Table 2: Primary seed formulation (Solid W/V, formulation volume 2L, supplemented with purified water**

| Material | Yeast powder | Polypeptone | Glucose |
|---|---|---|---|
| Ratio | 1.0% | 2.0% | 2.0% |

② Secondary seed culturing

Secondary seed inoculation: Under sterile conditions, the primary seed solution was transferred into a seed tank containing secondary culture medium, with an inoculation ratio of 5% to 11%.

The temperature of the seed tank was controlled to 30 ± 1 °C, with rotation speed≥ 100rpm (initial rotation speed of 100rpm) and tank pressure< 0.08MPa. The dissolved oxygen greater than 20% at this stage was ensured by controlling the rotation speed and ventilation rate (initial ventilation rate of 20L/min). After 4 hours of culturing, samples were taken every 2 hours for microscopic examination, OD₆₀₀ was measured, and all parameters were recorded. The culturing was terminated when the OD₆₀₀ of the seed solution was 5-7, and the culture time was 10± 4h.

**Table 3 Secondary Seed Formulation (Solid W/V, formulation volume 2L, supplemented with purified water)**

| Component | Polypeptone | Yeast powder | Glucose |
|---|---|---|---|
| Content | 2.0% | 1.0% | 2.0% |

### ③ Fermentation culturing

When the OD₆₀₀ of the secondary seed solution was between 5-7, the original basic fermentation culture medium (4ml/L) was added to the fermentation tank under sterile conditions, and the secondary seed solution was transferred into a 50L fermentation tank under sterile conditions. The temperature was controlled to 30 ± 1 °C, with pH 5.0 ± 0.2 (ammonia water) and tank pressure<0.08MPa. The dissolved oxygen greater than 20% at this stage was ensured by controlling the rotation speed (initial rotation speed of 100rpm), ventilation rate (initial ventilation rate of 100L/min), and oxygen flow rate. When DO rapidly increased, this stage was completed, and the wet weight of the bacteria at this stage was 90-150 g/L, requiring about 16-24 hours. The components of the original basic fermentation culture medium were: K₂SO₄: 0.91%; MgSO₄: 0.36%; CaSO₄·2H₂O: 0.059%; 85% H₃PO₄: 2.5% (V/V); KOH: 0.206%; Glycerol: 4%; GPE: 0.05% -0.1%; and PTM₁: 0.4% (V/V).

Methanol induction stage: the temperature was controlled to 20-30°C, with pH 6.0 + 0.2 (ammonia water) and tank pressure<0.08MPa. The dissolved oxygen greater than 20% at this stage was ensured by controlling the methanol replenishment speed, rotation speed, ventilation rate, and oxygen flow rate. The initial amount of methanol added should not exceed 0.5% of the initial fermentation volume. When the dissolved oxygen rapidly increased to its peak (usually within 1 minute), methanol should be added at a constant speed and the flow rate should be gradually increased. The maximum flow rate of methanol should not exceed 12mL/h.L (12mL/hour supplement per liter of fermentation broth) volume of the fermentation broth. If DO could not be maintained above 20%, stop the addition of methanol solution, wait until the DO reached its peak, and then resume the addition of methanol solution. The wet weight of the bacteria at this stage was 350-600g/L, and the methanol induction time should not be less than 60h. The components of the original methanol induced culture medium were: 100% methanol+12mL/L PTM1.

At different stages after methanol induction, the supernatant of the induction culture medium was taken to detect the thrombin activity of expressed batroxobin according to the method in Example 2. The fermentation results were shown in Fig. 2. It can be seen from the dtermination results in Fig. 2 that in small-scale fermentation, the methanol induction time was around 60 hours, and the activity of recombinant batroxobin in the culture medium reached its highest level. After 60 hours, the activity of batroxobin tended to stabilize, but showed a slight decrease, which was speculated to be due to the enhanced degradation of the recombinant enzyme by impurity proteins in the medium.

### 2) Optimization of the components of the basic fermentation culture medium for fermentation culturing

The components of the original basic fermentation culture medium in the above " ③ Fermentation culturing" were optimized, that is, the optimized basic fermentation culture medium (4ml/L) was added to the fermentation tank after transferring the secondary seed solution, and other fermentation conditions were the same as in 1) above. The components of the optimized basic fermentation culture medium are: K₂SO₄: 0.91%; MgSO₄: 0.36%; CaSO₄·2H₂O: 0.059%; 85% H₃PO₄: 2.5% (V/V); KOH: 0.206%; Glycerol: 4%; GPE: 0.05%-0.1%; PTM₁: 0.4% (V/V) and casamino acids: 0.5%.

At different stages after methanol induction, the supernatant of the induction culture medium was taken to detect the thrombin activity of expressed batroxobin according to the method in Example 2. The fermentation results were shown in Fig. 3. It can be seen from the measurement results in Fig. 3 that by optimizing the components of the basic fermentation culture medium, the activity of recombinant batroxobin in the culture medium had been improved to some extent at various stages of methanol induction.

### 3) Optimization of the components of the methanol induced culture medium for fermentation culturing

According to the fermentation induction method described in 2) above, the difference is that during the methanol induction stage, the components of the optimized methanol induced culture medium was used: (100% methanol+12mL/L PTM1) 95%+4.5% sorbitol+0.5% casamino acids, for induction.

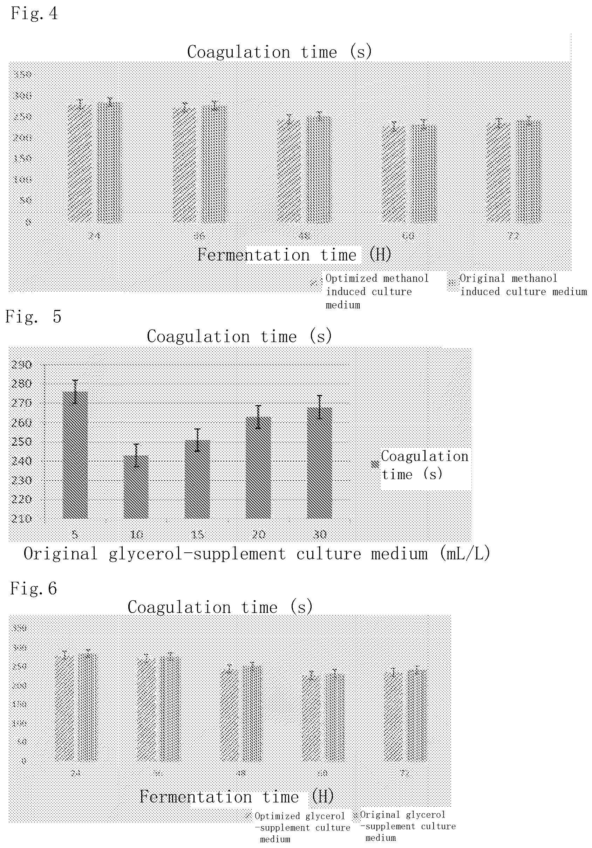

At different stages after methanol induction, the supernatant of the induction culture medium was taken to detect the thrombin activity of expressed batroxobin according to the method in Example 2. The fermentation results were shown in Fig. 4. It can be seen from the determination results in Fig. 4 that by optimizing the components of the methanol induced culture medium, the activity of recombinant batroxobin in the culture medium had been improved to some extent at various stages of induction.

### 4) The effect of the step ofadding glycerol supplement steps on the activity of fermented proteins

The initial stage of bacteria fermentation required a huge amount of carbon sources, nitrogen sources, and inorganic salts in the culture medium. Therefore, appropriate supplements should be given in the early stage of fermentation and the nutrient ingredients in the culture medium should be increased to improve the expression level of the recombinant protein.

According to the fermentation induction method in 3) above, the difference is that in the initial stage of bacteria fermentation, the temperature was controlled at 30 ± 1 °C, with pH 5.0 ± 0.2 and tank pressure<0.08MPa. It was cultured for about 4 hours. Different volumes (5mL, 10mL, 15mL, 20mL, 30mL) of the original glycerol-supplement culture medium were added to each liter of fermentation culture medium. The components of the original glycerol-supplement culture medium were: 50% glycerol+12mL/L PTM1.

After about 60 hours of methanol induction, the supernatant of the induction culture medium was taken and the thrombin activity of the expressed batroxobin was detected according to the method in Example 2. The fermentation results were shown in Fig. 5. It can be seen from the determination results in Fig. 5 that the activity of methanol induced fermentation proteins had been improved to a certain extent through the step of supplementing glycerol. The increase in the volume of glycerol from 5mL to 10mL in the original glycerol-supplement culture medium resulted in a significant increase in the activity of batroxobin. Although the amount of glycerol was further increased on this basis, the activity did not show a significant improvement.

### 5) The effect of optimizing the glycerol-supplement culture medium on the activity of fermented proteins

According to the fermentation induction method described in 4) above, the difference is that the added glycerol-supplement culture medium was optimized and the supernatant was taken at different stages of induction.

At the initial stage of bacteria fermentation, the temperature was controlled at 30 ± 1 °C, with pH 5.0 ± 0.2 and tank pressure<0.08MPa. It was cultured for about 4 hours. 10mL of optimized glycerol-supplement culture medium was added to per liter of fermentation culture medium. The components of the optimized glycerol-supplement culture medium were: 50% glycerol+12mL/L PTM1+0.5% casamino acids.

At different stages after induction, the supernatant of the induction culture medium was taken to detect the thrombin activity of expressed batroxobin according to the method in Example 2. The fermentation results were shown in Fig. 6. It can be seen from the determination results in Fig. 6 that by the step of optimizing the glycerol-supplement culture medium, the activity of fermented proteins induced by methanol has been improved to a certain extent.

### 6) Induction under different pH conditions of fermentation culture medium

According to the fermentation induction method described in 5) above, the difference was that the initial pH value of the fermentation culture medium was adjusted. The pH value of the fermentation culture medium was adjusted to 5.0, 5.5, 6.0, 6.5, and 7.0, respectively, and the activity of recombinant batroxobin in the medium was detected after methanol induction for about 60 hours.

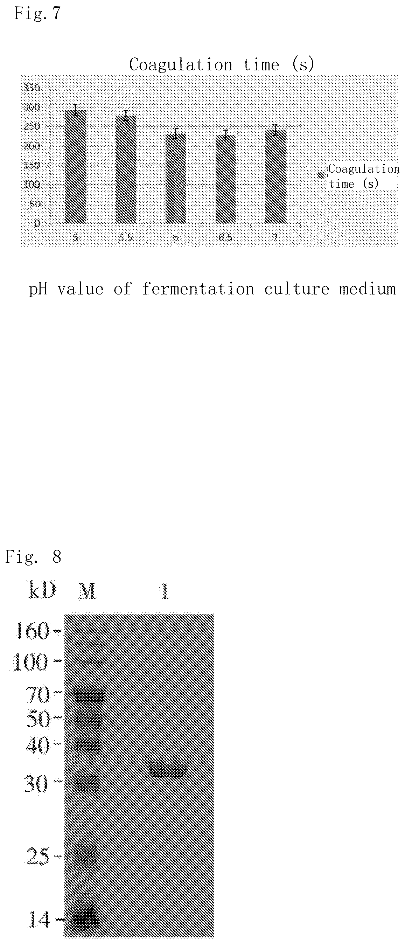

At different stages after methanol induction, the supernatant of the induction culture medium was taken to detect the thrombin activity of expressed batroxobin according to the method in Example 2. The fermentation results were shown in Fig. 7. It can be seen from the determination results in Fig. 7 that adjusting the pH value of the fermentation culture medium had an effect on the activity of methanol induced fermentation proteins. Among them, at a pH value of 6.5, the fermented recombinant protein exhibited higher activity, while a lower pH value actually had an inhibitory effect on the expression activity of the protein.

### Example 4. Fermentation Purification of Yeast X-33/pPICZαA-Batr-2

The fermentation according to condition 6) in Example 3 (pH value selected as 6.5) was collected and the supernatant of the culture medium was purified according to the following method.
1) The supernatant of yeast fermentation broth containing batroxobin was taken and the pH value and conductivity were adjusted; 2) Perform chromatography over cationic resin; the elution operation of chromatography was to remove impurity proteins with low salt buffer, to elute and remove pigments by using buffer PB with a pH adjusted to 8.0, and finally to elute the target protein by using buffer Tris-HCl with a pH of 9.0. 3) Perform chromatography over anion resin; the elution buffer is 50mM Tris HCl+0.5M NaCl, pH 9.0. 4) Unidirectional tangential-flow filtration and concentration; 5) the concentrated protein solution is subjected to gel filtration chromatography, and the elution buffer is 20mM PB+0.15M NaCl, pH6.0. 6) The target protein peak was eluted and collected to obtain the purified batroxobin. The purity of the purified recombinant batroxobin was detected by SDS electrophoresis, and the results were shown in Fig. 8.

### Example 5. Determination of enzyme activity between purified recombinant batroxobin and commercially available natural batroxobin

Commercially available batroxobin was taken and dissolved in buffer (20mM PB+0.15M NaCl, pH 6.0), and the protein concentration measured with conventional methods was consistent with the purified recombinant batroxobin prepared by the method in Example 4.

The method for determining enzyme activity refers to the method in Example 2: the method for determining thrombin activity was as follows:
0.2ml of human-citrate coagulation quality control plasma was taken and added to a 96-well plate, incubated at 37 °C for 3 minutes. 0.2ml of sample solution preheated at 37 °C was added, immediately shaken to mix evenly, and timed, and the plasma coagulation condition was begun to check at 40s. The initial coagulation time was recorded, and 3 tubes were determined simultaneously. The error should be less than 20s. If the initial coagulation time was less than 40s, the concentration of the test sample solution that coagulated within (60 ± 20) seconds was recorded, when the test sample solution was diluted several times. Under the above conditions, the amount of enzyme that could coagulate 0.2ml of human-citrate anticoagulant plasma in 60s was defined as one enzyme activity unit. The determining results were shown in Table 4.

**Table 4: Enzyme activity determination of recombinant batroxobin**

| Enzyme type | Recombinant batroxobin | Commercially available batroxobin |
|---|---|---|
| Coagulation time (S) | 183 | 174 |

The present invention illustrates the process method of the present invention via the above examples, but the present invention is not limited to the above process steps, that is, it does not mean that the present invention must rely on the above process steps to be implemented. Those skilled in the art should understand that any improvement to the present invention, equivalent substitution of raw materials used in the present invention, addition of auxiliary ingredients, selection of specific manners, etc., are all fallen into the scope of protection and disclosure of the present invention.

## Claims

1. A culture medium for recombinant batroxobin fermentation, comprising: optimized basic fermentation culture medium, optimized glycerol-supplement culture medium, and optimized methanol induced culture medium, **characterized in that** the components of the optimized basic fermentation culture medium are: K₂SO₄: 0.91%, MgSO₄: 0.36%, CaSO₄•2H₂O: 0.059%, 85% H₃PO₄: 2.5% (V/V), KOH: 0.206%, glycerol: 4%, GPE: 0.05%-0.1%, PTM1: 0.4% (V/V), and casamino acids: 0.5%; the components of the optimized glycerol-supplement culture medium are: 50% glycerol + 12mL/L PTM1 + 0.5% casamino acids; and the components of the optimized methanol induced culture medium are: 95% (100% methanol + 12mL/L PTM1) + 4.5% sorbitol + 0.5% casamino acids.

2. A fermentation method for batroxobin, **characterized by** comprising the following steps:
1) Primary seed culturing, inoculating a preserved strain into a primary seed culture medium, wherein the formulation for the primary seed culture medium is yeast powder, 1.0%; polypeptone 2.0%; glucose, 2.0%, formulated with purified water and sterilized for future use;
2) Secondary seed culturing, under sterile conditions, inoculating the primary seed solution into a seed tank containing a secondary culture medium, with an inoculation ratio of 5%-11%, the formulation for the secondary seed culture medium is yeast powder, 1.0%; polypeptone 2.0%; glucose, 2.0%, formulated with purified water and sterilized for future use;
3) Fermentation culturing, when the OD600 of the secondary seed solution is between 5-7, adding the optimized basic fermentation culture medium (4ml/L) to a fermentation tank under sterile conditions, transferring the secondary seed solution into the fermentation tank under sterile conditions, and after incubation for 4 hours, adding the optimized glycerol-supplement culture medium to the fermentation tank, adjusting pH value, and inducing with methanol; and
4) Collecting and purifying the supernatant to obtain recombinant batroxobin.

3. The fermentation method according to claim 2, **characterized in that** the components of the optimized basic fermentation culture medium are: K₂SO₄: 0.91%, MgSO₄: 0.36%, CaSO₄•2H₂O: 0.059%, 85% H₃PO₄: 2.5% (V/V), KOH: 0.206%, glycerol: 4%, GPE: 0.05%-0.1%, PTM1: 0.4% (V/V), and casamino acids: 0.5%.

4. The fermentation method according to claim 2, **characterized in that** the components of the optimized glycerol-supplement culture medium are: 95% (100% methanol + 12mL/L PTM1) + 4.5% sorbitol + 0.5% casamino acids.

5. The fermentation method according to claim 2, **characterized in that** during the methanol induction stage, the optimized methanol induced culture medium is used for induction, and the components of the optimized methanol induced culture medium are: 95% (100% methanol + 12mL/L PTM1) + 4.5% sorbitol + 0.5% casamino acids.

6. The fermentation method according to claim 2, **characterized in that** the adjusted pH range is 5.0-7.0.

7. The fermentation method according to claim 2, **characterized in that** the formulation of the PTM1 is: 0.6% CuSO₄ ▪ 5H₂O, 0.008% NaI, 0.3% MnSO₄ ▪ H₂O, 0.02% Na₂MoO₄ ▪ 2H₂O, 0.02% H₃BO₃, 0.05% CoCl₂ ▪ 6H₂O, 6.5% FeSO₄ ▪ 7H₂O, 2% ZnCl₂, 0.02% Biotin, and 0.0005% (V/V) H₂SO₄.
